(19) Europäisches Patentamt / European Patent Office / Office européen des brevets

(11) **EP 4 268 794 A1**

(12) **EUROPEAN PATENT APPLICATION**
published in accordance with Art. 153(4) EPC

(43) Date of publication:
**01.11.2023 Bulletin 2023/44**

(21) Application number: **21915338.4**

(22) Date of filing: **28.12.2021**

(51) International Patent Classification (IPC):
**A61K 6/853** (2020.01)     **A61K 6/864** (2020.01)

(52) Cooperative Patent Classification (CPC):
**A61K 6/853; A61K 6/864**

(86) International application number:
**PCT/JP2021/048984**

(87) International publication number:
**WO 2022/145480 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2020 JP 2020219718**

(71) Applicant: **Kuraray Noritake Dental Inc.**
**Kurashiki-shi, Okayama 710-0801 (JP)**

(72) Inventor: **FUJIMAKI, Ryo**
**Tainai-shi, Niigata 959-2653 (JP)**

(74) Representative: **D Young & Co LLP**
**120 Holborn**
**London EC1N 2DY (GB)**

(54) **CURABLE CALCIUM PHOSPHATE DENTAL CEMENT**

(57)  The present invention provides curable calcium phosphate dental cement that has excellent sealability for dentinal tubule, is expected to form fluoroapatite, and has a curing time that is advantageous for clinical use. The present invention is directed to curable calcium phosphate dental cement including: a first material as powder or nonaqueous paste; and a second material as liquid or aqueous paste, and, in the curable calcium phosphate dental cement, the first material comprises tetracalcium phosphate (A), alkali metal salt of phosphoric acid (B), and acidic calcium phosphate (C), the second material comprises water (D), and at least one of the first material and the second material comprises a fluorine compound (E) and an organic acid (F) having a molecular weight of 10000 or less.

EP 4 268 794 A1

**Description**

TECHNICAL FIELD

The present invention relates to curable calcium phosphate dental cement.

BACKGROUND ART

Along with a so-called 8020 campaign (improvement in oral hygiene, preservation of dentin (MI: Minimal Intervention)) for attempting to keep 20 or more teeth of their own even at the age of 80, the tooth survival rate of elderly people has been rapidly enhanced. However, this causes a problem with exposure of dentin due to new dental diseases (for example, wear of tooth, alveolar bone loss due to periodontal disease). In the exposed dentin, a mineral concentration of tissue constituting the dentin is low unlike in enamel, and dental caries resistance is not good, and, furthermore, dentinal tubule having a diameter of about 1 $\mu$m or less to about 3 $\mu$m is opened due to action of acid in an oral cavity to cause, for example, onset of hypersensitivity. As a method for improving this, a method for covering with a polymer-based material, and a method in which two kinds of materials are alternately applied, inorganic salt is deposited, and a physical barrier is formed to seal the dentinal tubule, have been known. However, in these methods, a shallow portion near the opening of the dentinal tubule or the surface thereof is merely covered, and breakage is easily caused by abrasion due to a toothbrush or the like. Although the material is highly biocompatible, a problem arises that application of the material causes adhesion of plaque, and inflammation or root surface dental caries is caused.

Meanwhile, as a curable calcium phosphate composition, calcium phosphate cement in which tetracalcium phosphate and dicalcium phosphate anhydrous are contained in combination, has been known. The calcium phosphate cement is gradually converted to bioabsorbable hydroxyapatite ($Ca_{10}(PO_4)_6(OH)_2$) in a living body or an oral cavity, and can be further united with living body hard tissue while the morphology is maintained. As one of examples in which such calcium phosphate cement is used for sealing dentinal tubule, Patent Literature 1 discloses a dentin remineralizing agent containing a specific amount of alkali metal salt of phosphoric acid in tetracalcium phosphate particles, and indicates that a dense hydroxyapatite layer is formed on the dentin surface, and hydroxyapatite is deposited also up to a deep portion of the dentinal tubule, to seal the dentinal tubule.

Furthermore, Patent Literature 1 discloses calcium phosphate cement containing a fluorine compound, and indicates that, since the fluorine compound is contained, acid resistance is imparted to dentin, and mineralization is also promoted. As a specific fluorine compound, sodium fluoride is used, and it is suggested that fluoroapatite is formed to impart acid resistance to dentin.

For such calcium phosphate cement, operationality in clinical use as well as the above-described clinical efficacy is very important. Specifically, in general, the calcium phosphate cement is produced and used by mixing two materials, which are a powder material and a liquid material, immediately before it is used, and, in a case where a curing time from the start of the mixing to the end of curing is excessively long, the calcium phosphate cement needs to be held until a cured product is settled at a desired position, and this is not practically preferable. Meanwhile, in a case where the curing time is short, curing progresses during a kneading operation, so that a uniformly kneaded body cannot be obtained, a curing reaction is non-uniformly made, and strength may be locally reduced in a cured body of the calcium phosphate cement.

Furthermore, Patent Literature 1 specifically indicates that disodium hydrogen phosphate ($Na_2HPO_4$) is added as alkali metal salt of phosphoric acid, and an effect of enhancing sealability for dentinal tubule also in a deep portion of the dentinal tubule is thus exhibited.

Furthermore, Patent Literature 2 discloses calcium phosphate cement containing an organic acid (for example, succinic acid) or salt of an organic acid, and indicates that an operation time is increased as compared with calcium phosphate cement that does not contain an organic acid or salt of an organic acid, that is, the curing time is delayed.

Patent Literature 3 discloses a composition of matter for dental restoration and implants and restoration, the composition including a powdery calcium compound containing one kind or a plurality of kinds of specific calcium phosphate salts, and a specific acidic calcium phosphate solution. Furthermore, Patent Literature 3 also indicates that disodium phosphate (disodium hydrogen phosphate; $Na_2HPO_4$) is blended with cement paste as a pore forming material that can form a diameter (about 30 to 500 $\mu$m) effective for causing neovascularization. Moreover, Patent Literature 3 indicates that, in a case where sodium fluoride is mixed with powder of calcium phosphate cement, concentrations of free anion component and calcium in a solution are reduced as a result of generation or deposition of a complex, a phosphate ion concentration is relatively increased, and the increase of the phosphate ion concentration promotes cement setting reaction, that is, the curing time becomes short when sodium fluoride is contained.

CITATION LIST

Patent Literature

Patent Literature 1: WO2010/113800
Patent Literature 2: US Patent No. 8557038
Patent Literature 3: JP 2007-522113 A

SUMMARY OF INVENTION

Technical Problem

However, according to examination made by the inventor of the present invention, in Patent Literature 1, the curing time of the calcium phosphate cement containing a fluorine compound is long, and the curing time cannot be shortened to a certain time or shorter even by adjusting an amount of alkali metal salt of phosphoric acid (for example, disodium hydrogen phosphate), and there is room for further improvement.

According to the disclosure of Patent Literature 2, since the calcium phosphate cement does not contain a fluorine compound, formation of fluoroapatite for imparting acid resistance to dentin cannot be expected, and, furthermore, the curing time is delayed since an organic acid or salt thereof is contained, and it is also indicated in examples that, in a case where a concentration of citric acid is increased, the operation time is also increased.

The inventor of the present invention has found through examination that a cured product of the calcium phosphate cement of Patent Literature 3 does not become dense, and sealability for dentinal tubule is reduced. Moreover, it has been found that, in a case where disodium hydrogen phosphate ($Na_2HPO_4$) is simply added to the calcium phosphate cement of Patent Literature 3, the curing time is delayed.

The present invention has been made in order to solve the aforementioned problem, and an object of the present invention is to provide curable calcium phosphate dental cement that has excellent sealability for dentinal tubule, is expected to form fluoroapatite, and has a curing time that is advantageous for clinical use.

Solution to Problem

The inventor of the present invention has found, as a result of thorough examination, that the aforementioned problem can be solved by a composition obtained by adding a specific organic acid to curable calcium phosphate dental cement including tetracalcium phosphate particles, particles of alkali metal salt of phosphoric acid, acidic calcium phosphate particles, water, and a fluorine compound, and has made further examination to complete the present invention.

That is, the present invention comprises the following inventions.

[1] Curable calcium phosphate dental cement including: a first material as powder or nonaqueous paste; and a second material as liquid or aqueous paste, in which

the first material comprises tetracalcium phosphate (A), alkali metal salt of phosphoric acid (B), and acidic calcium phosphate (C),
the second material comprises water (D), and
at least one of the first material and the second material comprises a fluorine compound (E) and an organic acid (F) having a molecular weight of 10000 or less.

[2] The curable calcium phosphate dental cement according to [1], in which the fluorine compound (E) is at least one selected from the group consisting of sodium fluoride, potassium fluoride, sodium monofluorophosphate, and stannous fluoride.

[3] The curable calcium phosphate dental cement according to [1] or [2], in which the fluorine compound (E) is sodium fluoride or potassium fluoride.

[4] The curable calcium phosphate dental cement according to any one of [1] to [3], in which an amount of the fluorine compound (E) in terms of fluoride ions is 0.01 to 3% of an entire amount of the curable calcium phosphate dental cement.

[5] The curable calcium phosphate dental cement according to any one of [1] to [4], in which the organic acid (F) having a molecular weight of 10000 or less is a polyvalent organic acid having two or more acidic groups.

[6] The curable calcium phosphate dental cement according to any one of [1] to [5], in which the organic acid (F) having a molecular weight of 10000 or less is at least one selected from the group consisting of citric acid, malonic acid, succinic acid, oxalic acid, malic acid, and tartaric acid.

[7] The curable calcium phosphate dental cement according to any one of [1] to [6], in which the organic acid (F) having a molecular weight of 10000 or less is citric acid.

[8] The curable calcium phosphate dental cement according to any one of [1] to [7], in which a content of the organic acid (F) having a molecular weight of 10000 or less is greater than or equal to 0.005 parts by mass with respect to 100 parts by mass of the entire amount of the curable calcium phosphate dental cement.

[9] The curable calcium phosphate dental cement according to any one of [1] to [8], in which at least one of the first material and the second material further comprises salt of organic acid (G) having a molecular weight of 10000 or less.

[10] The curable calcium phosphate dental cement according to any one of [1] to [9], in which at least one of the first material and the second material further comprises at least one selected from the group consisting of metal oxide particles and light anhydrous silicic acid particles which have an average particle diameter of 0.002 to 20 $\mu$m.

[11] The curable calcium phosphate dental cement according to any one of [1] to [10], in which the second material has a pH of greater than or equal to 6.0.

[12] The curable calcium phosphate dental cement according to any one of [1] to [11], in which paste immediately after malaxation of the first material and the second material has a pH of greater than or equal to 5.5.

[13] The curable calcium phosphate dental cement according to any one of [1] to [12], in which the second material comprises the fluorine compound (E), and the organic acid (F) having a molecular weight of 10000 or less.

[14] The curable calcium phosphate dental cement according to any one of [1] to [13], in which the first material is powder, and the second material is liquid.

[15] The curable calcium phosphate dental cement according to any one of [1] to [14], in which the tetracalcium phosphate (A) is in a form of particles, and has an average particle diameter of 0.5 to 40 $\mu$m.

[16] The curable calcium phosphate dental cement according to any one of [1] to [15], in which the alkali metal salt of phosphoric acid (B) is in a form of particles, and has an average particle diameter of 0.5 to 20 $\mu$m.

[17] The curable calcium phosphate dental cement according to any one of [1] to [16], in which the acidic calcium phosphate (C) is in a form of particles, and has an average particle diameter of 0.1 to 7 $\mu$m.

[18] Dentifrice comprising the curable calcium phosphate dental cement according to any one of [1] to [17].

[19] A dentinal hypersensitivity inhibitor comprising the curable calcium phosphate dental cement according to any one of [1] to [17].

Advantageous Effects of Invention

The present invention can provide curable calcium phosphate dental cement that has excellent sealability for dentinal tubule, is expected to form fluoroapatite, and has a curing time that is advantageous for clinical use.

DESCRIPTION OF EMBODIMENTS

The present invention will be described below in detail. Curable calcium phosphate dental cement of the present invention is formed of a first material that is powder or nonaqueous paste and a second material that is liquid or aqueous paste. The first material comprises tetracalcium phosphate (A), alkali metal salt of phosphoric acid (B), and acidic calcium phosphate (C). The second material comprises water (D). At least one of the first material or the second material comprises a fluorine compound (E) and an organic acid (F) having a molecular weight of 10000 or less. The "nonaqueous" of the "nonaqueous paste" means paste in which a water content in the paste is less than or equal to 5%, preferably less than or equal to 3%, and more preferably less than or equal to 1%.

The curable calcium phosphate dental cement having the above-described configuration has excellent sealability for dentinal tubule, is expected to form fluoroapatite, and has a curing time that is advantageous for clinical use.

In the present specification, the upper limit value and the lower limit value of a numerical value range (content of each component and the like) can be combined as appropriate.

Although the present invention is not limited in any way, the reason why the excellent effects as described above are exhibited is considered as follows. Firstly, in a case where a fluorine compound is introduced in calcium phosphate cement including tetracalcium phosphate, alkali metal salt of phosphoric acid, and acidic calcium phosphate, a curing time is significantly delayed. This is because it is estimated that, in a state where calcium ions are insufficient with respect to phosphate ions, calcium fluoride is deposited by reaction between calcium ions and fluoride ions which is generated when a calcium compound and water are mixed, an amount of calcium ions in the system is thus reduced, and an ion ratio becomes unbalanced and deviates from an ion balance suitable for a curing reaction. The curable calcium phosphate dental cement of the present invention further comprises a specific organic acid. Thus, it is considered that the deposited calcium fluoride is dissolved by the organic acid. As a result, an ion ratio becomes suitable for a curing reaction without reducing a concentration of calcium ions in the system. Therefore, it is estimated that, even in a case where a fluorine compound is contained, the curable calcium phosphate dental cement having a suitable curing time can be obtained. Furthermore, it is estimated, as one of the reasons, that a fluorine compound reacts with the organic acid before reacting with calcium ions, to form a complex of the fluorine compound and the organic acid, generation of calcium fluoride is thus delayed when the fluorine compound and the calcium salt are mixed, and this can also shorten the curing time.

Tetracalcium phosphate (A)

The curable calcium phosphate dental cement of the present invention comprises tetracalcium phosphate $(Ca_4(PO_4)_2O; TTCP)$ (A) in powder or nonaqueous paste as the first material. The tetracalcium phosphate (A) is preferably in the form of particles. The average particle diameter of the tetracalcium phosphate (A) particles is not particularly limited. However, in a case where the average particle diameter is greater than or equal to 0.5 $\mu$m, the tetracalcium phosphate (A) is not excessively dissolved, and, thus, a pH in an aqueous solution is not excessively high, and hydroxyapatite is smoothly deposited. Therefore, the average particle diameter of the tetracalcium phosphate (A) particles is preferably greater than or equal to 0.5 $\mu$m, more preferably greater than or equal to 0.7 $\mu$m, and even more preferably greater than or equal to 1 $\mu$m. Meanwhile, in a case where the average particle diameter is less than or equal to 40 $\mu$m, paste obtained by mixing with liquid or aqueous paste as the second material described below has a sufficient viscosity and thus has preferable paste characteristics. Furthermore, feeling of roughness is reduced during malaxation of paste, and advantageous operationality can be maintained. Therefore, the average particle diameter of the tetracalcium phosphate (A) is preferably less than or equal to 40 $\mu$m, more preferably less than or equal to 20 $\mu$m, and even more preferably less than or equal to 10 $\mu$m. In the description herein, the average particle diameter of the tetracalcium phosphate (A) particles used in the present invention is measured by using a laser diffraction type particle size distribution analyzer, and calculated. For example, the average particle diameter can be calculated by a method indicated below in Examples.
A content of the tetracalcium phosphate (A) in the curable calcium phosphate dental cement of the present invention is, but is not particularly limited to, preferably 1 to 80 parts by mass, more preferably 1 to 70 parts by mass, and even more preferably 1 to 60 parts by mass with respect to 100 parts by mass of the total content of the curable calcium phosphate dental cement. In a case where the content of the tetracalcium phosphate (A) is greater than or equal to 1 part by mass, the curable calcium phosphate dental cement has enhanced sealability for dentinal tubule. Meanwhile, in a case where the content of the tetracalcium phosphate (A) is less than or equal to 80 parts by mass, the curable calcium phosphate dental cement has a viscosity in an appropriate range, and paste having good operationality can be obtained.
A method for manufacturing the tetracalcium phosphate (A) to be used in the present invention is not particularly limited. Commercially available tetracalcium phosphate particles may be used as they are or by adjusting particle diameters as appropriate by pulverization. As a pulverizing method, a method that is the same as a method for pulverizing the acidic calcium phosphate (C) described below can be adopted.

·Alkali metal salt of phosphoric acid (B)

The curable calcium phosphate dental cement of the present invention comprises the alkali metal salt of phosphoric acid (B) in powder or nonaqueous paste as the first material. The alkali metal salt of phosphoric acid (B) is preferably in the form of particles. The alkali metal salt of phosphoric acid (B) is not particularly limited, and examples thereof include disodium hydrogen phosphate $(Na_2HPO_4)$, dipotassium hydrogen phosphate $(K_2HPO_4)$, lithium dihydrogen phosphate $(LiH_2PO_4)$, sodium dihydrogen phosphate $(NaH_2PO_4; MSP)$, potassium dihydrogen phosphate $(KH_2PO_4; KDP)$, trisodium phosphate $(Na_3PO_4; TSP)$, and tripotassium phosphate $(K_3PO_4)$. One of them may be used, or two or more of them may be used in combination. Among them, the alkali metal salt of phosphoric acid (B) is preferably disodium hydrogen phosphate and/or sodium dihydrogen phosphate from the viewpoint of safety and easiness of obtaining a material having high purity. From the viewpoint of safety, alkali metal ions in the alkali metal salt of phosphoric acid (B) to be used in the present invention are preferably sodium ions.
The average particle diameter of particles of the alkali metal salt of phosphoric acid (B) to be used in the present invention is not particularly limited. However, in a case where the average particle diameter of particles of the alkali metal salt of phosphoric acid (B) is greater than or equal to 0.5 $\mu$m, significant aggregation is not caused and the particles can be uniformly dispersed in paste or powder, and, when the curable calcium phosphate dental cement of the present invention is converted to hydroxyapatite, holes are not generated in the hydroxyapatite and a high dentinal tubule sealing rate can be maintained. Therefore, the average particle diameter of particles of the alkali metal salt of phosphoric acid (B) is preferably greater than or equal to 0.5 $\mu$m, more preferably greater than or equal to 4 $\mu$m, and even more preferably greater than or equal to 8 $\mu$m. Meanwhile, in a case where the average particle diameter of particles of the alkali metal salt of phosphoric acid (B) is less than or equal to 20 $\mu$m, when the curable calcium phosphate dental cement of the present invention is converted to hydroxyapatite, holes are not generated in the hydroxyapatite, a high dentinal tubule sealing rate can be maintained, and hypersensitivity can be inhibited. Furthermore, it is possible to prevent increase of feeling of roughness and reduction of operationality as caused due to the alkali metal salt of phosphoric acid (B) which has not been dissolved being left in the paste when the cement is applied onto a dentin surface so as to rub the paste for inhibiting dentinal hypersensitivity. Therefore, the average particle diameter of the alkali metal salt of phosphoric acid (B) is preferably less than or equal to 20 $\mu$m, more preferably less than or equal to 16 $\mu$m, and even more preferably less than or equal to 12 $\mu$m. The average particle diameter of particles of the alkali metal salt of phosphoric acid (B) can be calculated by a method that is similar to the method indicated for measuring the average particle diameter of the tetracalcium phosphate (A) particles.

The curable calcium phosphate dental cement of the present invention preferably comprises the alkali metal salt of phosphoric acid (B) in an amount of 5 to 98 parts by mass with respect to 100 parts by mass of the tetracalcium phosphate (A). Thus, since a certain amount of the alkali metal salt of phosphoric acid (B) as well as the tetracalcium phosphate (A) is contained, the curable calcium phosphate dental cement that allows hydroxyapatite to be more effectively deposited up to a deep portion of dentinal tubule can be provided. In a case where the content of the alkali metal salt of phosphoric acid (B) is less than 5 parts by mass, deposition of hydroxyapatite may be hindered. Therefore, the content of the alkali metal salt of phosphoric acid (B) is preferably greater than or equal to 5 parts by mass, more preferably greater than or equal to 7 parts by mass, and even more preferably greater than or equal to 10 parts by mass. Meanwhile, in a case where the content of the alkali metal salt of phosphoric acid (B) is greater than 98 parts by mass, deposition of hydroxyapatite may be hindered. Therefore, the content of the alkali metal salt of phosphoric acid (B) is preferably less than or equal to 98 parts by mass, more preferably less than or equal to 95 parts by mass, and even more preferably less than or equal to 90 parts by mass.

A method for manufacturing the alkali metal salt of phosphoric acid (B) to be used in the present invention is not particularly limited. Commercially available particles of alkali metal salt of phosphoric acid may be used as they are or by adjusting particle diameters as appropriate by pulverization. As a pulverizing method, a method that is the same as the method for pulverizing the acidic calcium phosphate (C) described below can be adopted.

·Acidic calcium phosphate (C)

The curable calcium phosphate dental cement of the present invention comprises the acidic calcium phosphate (C) in powder or nonaqueous paste as the first material. The acidic calcium phosphate (C) is preferably in the form of particles. In a case where the acidic calcium phosphate (C) is contained, a mineralizing effect can be further enhanced. The acidic calcium phosphate (C) to be used in the present invention is not particularly limited, and is preferably at least one selected from the group consisting of dicalcium phosphate anhydrous ($CaHPO_4$; DCPA), monocalcium phosphate anhydrous ($Ca(H_2PO_4)_2$), tricalcium phosphate ($Ca_3(PO_4)_2$; TCP), amorphous calcium phosphate ($Ca_3(PO_4)_2 \cdot nH_2O$, ACP), calcium dihydrogen pyrophosphate ($CaH_2P_2O_7$), dicalcium phosphate dihydrate ($CaHPO_4 \cdot 2H_2O$), and monocalcium phosphate monohydrate ($Ca(H_2PO_4)_2 \cdot H_2O$). As the acidic calcium phosphate (C), one of them may be used alone, or two or more of them may be used in combination. Tricalcium phosphate may be either $\alpha$-TCP or $\beta$-TCP. Among them, at least one selected from the group consisting of dicalcium phosphate anhydrous, monocalcium phosphate anhydrous, dicalcium phosphate dihydrate, and monocalcium phosphate monohydrate is more preferably used, and at least one selected from the group consisting of dicalcium phosphate anhydrous and monocalcium phosphate anhydrous is even more preferably used.

The average particle diameter of the acidic calcium phosphate (C) particles to be used in the present invention is not particularly limited. However, in a case where the average particle diameter is greater than or equal to 0.1 $\mu$m, unbalanced supply of calcium ions and phosphate ions due to dissolution in a liquid being excessively increased is prevented, and the viscosity of the paste obtained by mixing with a liquid can be appropriately maintained. The average particle diameter of the acidic calcium phosphate (C) particles is preferably greater than or equal to 0.1 $\mu$m, more preferably greater than or equal to 0.5 $\mu$m, and even more preferably greater than or equal to 1 $\mu$m. Meanwhile, in a case where the average particle diameter is less than or equal to 7 $\mu$m, since the acidic calcium phosphate (C) is unlikely to be dissolved in a product obtained by malaxation of the first material and the second material, the tetracalcium phosphate (A) is prevented from being excessively dissolved. As a result, unbalanced supply of calcium ions and phosphate ions is prevented, a pH of an aqueous solution does not become excessively high, and hydroxyapatite is smoothly deposited. The average particle diameter of the acidic calcium phosphate (C) particles is preferably less than or equal to 7 $\mu$m, more preferably less than or equal to 5 $\mu$m, and even more preferably less than or equal to 3 $\mu$m. The average particle diameter of the acidic calcium phosphate (C) particles can be calculated by a method that is similar to the method indicated for measuring the average particle diameter of the tetracalcium phosphate (A) particles.

A method for manufacturing the acidic calcium phosphate (C) having such an average particle diameter is not particularly limited. A commercially available product may be used if available. However, preferably, a commercially available product is further pulverized and used in many cases. In this case, a pulverizing device such as a ball mill, a pestle and mortar machine, and a jet mill can be used. The acidic calcium phosphate (C) may be obtained by pulverizing acidic calcium phosphate material powder with a liquid medium such as alcohol by using a pestle and mortar machine, a ball mill, or the like to prepare a slurry, and drying the obtained slurry. At this time, a ball mill is preferably used as the pulverizing device, and alumina, zirconia, or the like is preferably used as the material of its pot and ball.

A blending ratio (A/C) of the tetracalcium phosphate (A) to the acidic calcium phosphate (C) is not particularly limited. A blending ratio in a range of a molar ratio of 40/60 to 60/40 is used. The above-described blending ratio (A/C) is preferably 45/55 to 55/45.

·Water (D)

In the curable calcium phosphate dental cement of the present invention, the second material is liquid or aqueous paste that contains water (D). In an embodiment, the second material is liquid that contains water (D) as a main component. The main component is a component having the largest content, and, for example, the content may be greater than or equal to 50% by mass, may be greater than or equal to 60% by mass, and may be greater than or equal to 70% by mass. The liquid that contains water (D) as the main component may be pure water, or a liquid that contains water (D) as the main component, and another component. Aqueous paste that contains water (D) as the main component represents a paste-like liquid that contains water (D) as the main component, and another component. The other component is not particularly limited, and examples thereof include polyhydric alcohols such as glycerin, ethylene glycol, propylene glycol, and diglycerin, sugar alcohols such as xylitol, sorbitol, and erythritol, and polyethers such as polyethylene glycol and polypropylene glycol.

·Fluorine compound (E)

In the curable calcium phosphate dental cement of the present invention, at least one of the first material or the second material comprises the fluorine compound (E). In a case where the fluorine compound (E) is included, fluoroapatite is formed, acid resistance can be imparted to dentin, and mineralization is promoted. In a preferable embodiment, from the viewpoint that calcium fluoride is not generated, and, thus, the amount of calcium ions in the system are not reduced, and a curing time is more unlikely to be delayed, for example, the curable calcium phosphate dental cement in which the second material comprises the fluorine compound (E) is used.

The fluorine compound (E) to be used in the present invention is not particularly limited, and examples thereof include sodium fluoride, potassium fluoride, ammonium fluoride, lithium fluoride, cesium fluoride, magnesium fluoride, calcium fluoride, strontium fluoride, barium fluoride, copper fluoride, zirconium fluoride, aluminum fluoride, stannous fluoride, sodium monofluorophosphate, potassium monofluorophosphorate, hydrofluoric acid, titanium sodium fluoride, titanium potassium fluoride, hexylamine hydrofluoride, laurylamine hydrofluoride, glycine hydrofluoride, alanine hydrofluoride, fluorosilanes, and diamine silver fluoride. Among them, sodium fluoride, potassium fluoride, sodium monofluorophosphate, and stannous fluoride are preferably used, and sodium fluoride and potassium fluoride are more preferably used. As the fluorine compound (E), one of them may be used alone, or two or more of them may be used in combination.

The content of the fluorine compound (E) is not particularly limited. An amount of the fluorine compound (E) in terms of fluoride ions is preferably 0.01 to 3%, more preferably 0.05 to 1%, and even more preferably 0.10 to 0.90% with respect to the entire amount of the curable calcium phosphate dental cement. In a case where the amount of the fluorine compound (E) in terms of fluoride ions is greater than 3%, safety may deteriorate, and the amount of the fluorine compound (E) in terms of fluoride ions is more preferably less than or equal to 1%. In a case where the amount of the fluorine compound (E) in terms of fluoride ions is greater than or equal to 0.01%, formation of fluoroapatite is expected. The amount of the fluorine compound (E) in terms of fluoride ions can be calculated by a method indicated below in Examples.

·Organic acid (F) having a molecular weight of 10000 or less

In the curable calcium phosphate dental cement of the present invention, at least one of the first material or the second material comprises the organic acid (F) having a molecular weight of 10000 or less (hereinafter, may be simply referred to as "organic acid (F)"). In a preferable embodiment, from the viewpoint that calcium fluoride is not generated, and, thus, the amount of calcium ions in the system are not reduced, and a curing time is more unlikely to be delayed since the organic acid (F) is included, for example, the curable calcium phosphate dental cement in which the second material comprises the organic acid (F) is used. Even if the fluorine compound (E) is blended in the curable calcium phosphate dental cement and calcium fluoride is formed, a concentration of calcium ions can be restored, and a curing time of the curable calcium phosphate dental cement of the present invention is consequently shortened, so that the curable calcium phosphate dental cement having a curing time that is advantageous for clinical use can be obtained. The molecular weight of the organic acid (F) of the present invention needs to be less than or equal to 10000. In a case where the molecular weight is greater than 10000, reaction with calcium ions causes paste to be in the form of high-viscosity gel after malaxation, and operationality is thus degraded. In addition thereto, it is estimated that, since solubility of organic acid itself having a molecular weight of greater than 10000 into water is poor, and solubility of calcium salt of organic acid having a molecular weight of greater than 10000 is low, the effect of the present invention is unlikely to be exhibited. The molecular weight of the organic acid (F) of the present invention is preferably less than or equal to 5000, and more preferably less than or equal to 2500 from the viewpoint of obtaining a more appropriate curing time. Although the lower limit of the molecular weight is not particularly limited, the molecular weight can be, for example, greater than or equal to 45 and may be greater than or equal to 100. In a case where the organic acid (F) is a compound having no polymer skeleton, the molecular weight represents the sum of atomic weights in a molecule. In a case where the organic acid (F) is a compound having a polymer skeleton, the molecular weight represents a weight-average molecular weight. The weight-average molecular weight represents a weight-average molecular weight in terms of polystyrene as determined by gel permeation chromatography.

As the organic acid (F) of the present invention, any organic acid having a molecular weight of 10000 or less can be used without any limitation, and examples thereof include an organic acid containing one or more acidic groups selected from, for example, a carboxyl group, a sulfonic acid group, and a phenol group. The organic acid (F) of the present invention is preferably a polyvalent organic acid from the viewpoint of solubility of calcium. The polyvalent organic acid represents an acid having two or more acidic groups, and specific examples thereof include an organic acid having a plurality of acidic groups selected from carboxyl groups, sulfonic acid groups, phenol groups, and the like, and an organic acid having a plurality of kinds of these acidic groups in combination. The acidic group is preferably a carboxyl group, a sulfonic acid group, and a phenol group, more preferably a carboxyl group and a sulfonic acid group, and even more preferably a carboxyl group. Examples of the polyvalent organic acid having a plurality of carboxyl groups include an organic acid (dicarboxylic acid) such as citric acid, malonic acid, succinic acid, oxalic acid, phthalic acid, malic acid, and tartaric acid having two carboxyl groups, and an organic acid such as diethylenetriamine pentaacetic acid (DTPA) and ethylenediaminetetraacetic acid (EDTA) having three or more carboxyl groups. As the organic acid (F), one of them may be used alone, or two or more of them may be used in combination. Examples of an organic acid having a carboxyl group and a phenol group in combination include gentisic acid. Among them, from the viewpoint of further shortening a curing time, a water-soluble organic acid is preferably used, at least one selected from citric acid, malonic acid, succinic acid, oxalic acid, malic acid, and tartaric acid is more preferably used, and citric acid is most preferably used.

The content of the organic acid (F) is preferably greater than or equal to 0.001 parts by mass, more preferably greater than or equal to 0.002 parts by mass, and even more preferably greater than or equal to 0.005 parts by mass with respect to 100 parts by mass of the total content of the curable calcium phosphate dental cement, from the viewpoint of shortening a curing time. From the viewpoint of, for example, sealability for dentinal tubule, storage stability, and operationality for the curable calcium phosphate dental cement, the content of the organic acid (F) is preferably less than or equal to 50 parts by mass, more preferably less than or equal to 30 parts by mass, and even more preferably less than or equal to 10 parts by mass.

In the curable calcium phosphate dental cement of the present invention, at least one of the first material or the second material comprises the fluorine compound (E) and the organic acid (F). An embodiment (X-1) is, for example, the curable calcium phosphate dental cement in which the first material comprises the fluorine compound (E) and the organic acid (F). Another embodiment (X-2) is, for example, the curable calcium phosphate dental cement in which the second material comprises the fluorine compound (E) and the organic acid (F). Another embodiment (X-3) is, for example, the curable calcium phosphate dental cement in which the first material comprises the fluorine compound (E) and the second material comprises the organic acid (F). An embodiment (X-4) is, for example, the curable calcium phosphate dental cement in which the first material comprises the organic acid (F) and the second material comprises the fluorine compound (E). Another embodiment (X-5) is, for example, the curable calcium phosphate dental cement in which the first material comprises the fluorine compound (E) and the organic acid (F) and the second material comprises the fluorine compound (E) and the organic acid (F). Another embodiment (X-6) is, for example, the curable calcium phosphate dental cement in which the first material comprises the fluorine compound (E) and the organic acid (F) and the second material comprises the organic acid (F). Another embodiment (X-7) is, for example, the curable calcium phosphate dental cement in which the first material comprises the fluorine compound (E) and the organic acid (F) and the second material comprises the fluorine compound (E). Another embodiment (X-8) is, for example, the curable calcium phosphate dental cement in which the first material comprises the fluorine compound (E) and the second material comprises the fluorine compound (E) and the organic acid (F). Another embodiment (X-9) is, for example, the curable calcium phosphate dental cement in which the first material comprises the organic acid (F) and the second material comprises the fluorine compound (E) and the organic acid (F).

·Salt of organic acid (G) having a molecular weight of 10000 or less

In the curable calcium phosphate dental cement of the present invention, at least one of the first material or the second material may include salt of organic acid (G) having a molecular weight of 10000 or less (hereinafter, may be simply referred to as "salt of organic acid (G)") as long as the effect of the present invention is not hindered. One kind of the salt of organic acid (G) may be used alone, or two or more kinds of the salt of organic acid (G) may be used in combination. The salt of organic acid (G) represents salt of the organic acid (F) (for example, alkali metal salt, alkaline-earth metal, ammonium salt) having a molecular weight of 10000 or less. The salt of organic acid (G) and the organic acid (F) are preferably contained in the same material. When the fluorine compound (E) and the organic acid (F) coexist, precipitate may be generated during storage. However, in a case where the salt of organic acid (G) is further contained, generation of the precipitate can be inhibited. The reason why the above-described problem is solved in a case where the salt of organic acid (G) coexists is uncertain. However, it is estimated that, in a case where the organic acid (F) and the salt of organic acid (G) coexist, the second material is in a neutral region, and deposition of acidic fluoride salt generated in an acidic state is inhibited. The content of the salt of organic acid (G) is preferably 0.1 to 50 parts by mass, more preferably 0.5 to 25 parts by mass, and even more preferably 1 to 10 parts by mass with respect to 100 parts by mass of calcium phosphate cement.

In a case where precipitate is generated while the second material is stored, since the second material needs to be shaken immediately before it is used, operationality is degraded. However, the second material can be used by shaking a container in which the second material is stored, and, therefore, the second material can be used as a material of the curable calcium phosphate dental cement of the present invention. Therefore, even if precipitate is generated, the effect of the present invention is not degraded.

A part of the salt of organic acid (G) is released in the first material, the second material, or paste obtained after malaxation, and is likely to exist as the organic acid (F). The total of the contents of the organic acid (F) having a molecular weight of 10000 or less and the salt of organic acid (G) having a molecular weight of 10000 or less is preferably greater than or equal to 0.001 parts by mass, more preferably greater than or equal to 0.01 parts by mass, and even more preferably greater than or equal to 0.1 parts by mass, with respect to 100 parts by mass of the total content of the curable calcium phosphate dental cement. From the viewpoint of sealability for dentinal tubule, storage stability, and operationality for the curable calcium phosphate dental cement, the total of the contents of the organic acid (F) and the salt of organic acid (G) is preferably less than or equal to 50 parts by mass, more preferably less than or equal to 30 parts by mass, and even more preferably less than or equal to 10 parts by mass, with respect to 100 parts by mass of the total content.

The curable calcium phosphate dental cement of the present invention may include a component other than the tetracalcium phosphate (A), the alkali metal salt of phosphoric acid (B), the acidic calcium phosphate (C), water (D), the fluorine compound (E), the organic acid (F) having a molecular weight of 10000 or less, and the salt of organic acid (G) having a molecular weight of 10000 or less as long as the effect of the present invention is not hindered. For example, a thickener (H) can be blended as necessary as long as the effect of the present invention is not affected.

Specific examples of the thickener (H) include polysaccharides such as carboxymethylcellulose, sodium carboxymethylcellulose, hydroxypropylcellulose, hydroxypropylmethylcellulose, polyvinyl alcohol, poly-L-lysin, poly-L-lysin salts, starch other than cellulose, carrageenan, guar gum, xanthan gum, cellulose gum, pectin, pectin salts, chitin, and chitosan; polyhydric alcohols such as glycerin, ethylene glycol, propylene glycol, and diglycerin; sugar alcohols such as xylitol, sorbitol, and erythritol; polyethers such as polyethylene glycol and polypropylene glycol; acidic polysaccharide esters such as propylene glycol alginate; proteins such as hyaluronic acid and salts thereof, collagen, gelatin, and derivatives thereof; polymers such as polyglutamic acid and salts thereof, polyaspartic acid and salts thereof, polystyrene sulfonic acid and salts thereof, and polyacrylic acid, and inorganic fillers typified by light anhydrous silicic acid, metal oxide, or the like. One kind of the thickener (H) may be used alone, or two or more kinds of the thickeners (H) may be used in combination. In a case where the thickener (H) is blended in paste, at least one selected from sodium carboxymethylcellulose, hydroxypropylcellulose, hydroxypropylmethylcellulose, and chitosan is preferable from the viewpoint of solubility in water and viscosity. The thickener (H) may be blended in the first material or may be blended in the second material. In a case where the thickener (H) is blended in powder, the inorganic filler is preferably contained, at least one selected from the group consisting of light anhydrous silicic acid particles and metal oxide particles having an average particle diameter of 0.002 to 20 $\mu$m is more preferably contained, and light anhydrous silicic acid particles having an average particle diameter of 0.002 to 20 $\mu$m is even more preferably contained.

Artificial sweetener such as aspartame, acesulfame potassium, liquorice extract, saccharin, and saccharin sodium may be added as necessary.

Moreover, for example, any pharmacologically acceptable drug can be blended. For example, antibacterial agents typified by cetyl pyridinium chloride etc., disinfectants, anticancer drugs, antibiotics, blood circulation improvers such as Actosin and PEG1, growth factors such as bFGF, PDGF, and BMP, and cells which promote hard tissue formation, such as osteoblasts, odontoblasts, and anaplastic bone marrow derived stem cells, embryonic stem (ES) cells, induced pluripotent stem (iPS) cells produced by dedifferentiating differentiated cells such as fibroblasts by gene introduction, and cells produced by differentiating the foregoing can be blended.

In the present invention, the paste-like curable calcium phosphate dental cement can be obtained through malaxation of powder or nonaqueous paste including the tetracalcium phosphate (A), the alkali metal salt of phosphoric acid (B), and the acidic calcium phosphate (C), and liquid or aqueous paste including water (D). The paste-like curable calcium phosphate dental cement containing water immediately starts reaction of converting to hydroxyapatite, and is thus prepared by malaxation immediately before it is used at a medical site. The malaxation operation is not particularly limited, and manual malaxation, malaxation using a static mixer, and the like are preferably adopted. However, since solubility of the alkali metal salt of phosphoric acid (B) with respect to water is not so high, the above-described malaxation method in which the alkali metal salt of phosphoric acid (B) is added as powder is preferably adopted. A solvent, other than water, which is used for the nonaqueous paste is not particularly limited, and examples thereof include polyhydric alcohols such as glycerin, ethylene glycol, propylene glycol, and diglycerin, and polyethers such as polyethylene glycol and polypropylene glycol.

In the present invention, a malaxation ratio between the first material and the second material directly affects operationality during malaxation, and can thus be set based on a mass ratio between the first material and water in the second material. A mixing ratio between the first material and the second material is not particularly limited. However, in a case where a mass ratio represented as the first material/water (D) in the second material is greater than or equal to 0.5, the paste is prevented from being excessively runny due to excessively high fluidity of the paste after malaxation. Therefore, the mass ratio is preferably greater than or equal to 0.5, more preferably greater than or equal to 0.8, and even more preferably greater than or equal to 1.1. Meanwhile, in a case where the mass ratio represented as the first material/water (D) in the second material is less than or equal to 1.9, the paste is prevented from being excessively hard due to excessively low fluidity of the paste after the malaxation. Therefore, the mass ratio is preferably less than or equal to 1.9, more preferably less than or equal to 1.8, even more preferably less than or equal to 1.6, and most preferably less than or equal to 1.3.

In the curable calcium phosphate dental cement of the present invention, powder containing the tetracalcium phosphate (A), the alkali metal salt of phosphoric acid (B), and the acidic calcium phosphate (C) is preferably mixed in advance. Thus, sealability for dentinal tubule becomes better, the hypersensitivity inhibiting effect is thus enhanced, and an effect as a dentinal hypersensitivity inhibitor is enhanced. For the above-described mixing, at least one selected from a jet mill, a pestle and mortar machine, a ball mill, a high-speed rotation mill, a planetary mill, a hybridizer, a mechanofusion machine, and a mixing extruder is preferably used. From the viewpoint of further enhancing sealability for dentinal tubule, at least one selected from a ball mill, a pestle and mortar machine, a high-speed rotation mill, and a jet mill is preferably used.

In a case where the second material of the present invention comprises the fluorine compound (E) and the organic acid (F), the curable calcium phosphate dental cement in which the second material further comprises the salt of organic acid (G) is preferable from the viewpoint of storage stability for the second material. High storage stability allows reduction of work of shaking a container that stores the second material before use, and operationality is enhanced. Meanwhile, even if storage stability is poor and precipitation is caused, the container that stores the second material can be shaken before use, and the effect other than the operationality is not degraded. It is estimated that the second material is likely to be maintained in a neutral region by the salt of organic acid (G), and deposition of acidic fluoride salt is thus inhibited. A pH of the second material is preferably greater than or equal to 3.0, more preferably greater than or equal to 4.0, even more preferably greater than or equal to 5.0, and most preferably greater than or equal to 6.0.

A pH of the curable calcium phosphate dental cement of the present invention (paste immediately after malaxation of the first material and the second material) is, but is not particularly limited to, preferably greater than or equal to 5.5, more preferably greater than or equal to 6.0, and even more preferably greater than or equal to 6.5 in order to prevent erosion of teeth. The upper limit value can be, but is not particularly limited to, for example, less than or equal to 11.

The curable calcium phosphate dental cement of the present invention is used in the form of paste. The curable calcium phosphate dental cement of the present invention may be packaged in a divided manner in product form. For example, the curable calcium phosphate dental cement of the present invention may be in the form of a kit as a product packaged in a divided manner as a combination of the first material and the second material.

The curable calcium phosphate dental cement of the present invention can be used as a filling restorative material used for filling a cavity or a lost part of dentin, a lining material, a luting material, a temporary sealing material, a root canal filling material, a temporary luting material, a coating material, a sealant material, dentifrice, a dentinal hypersensitivity inhibitor, and the like, and is particularly suitable as dentifrice and a dentinal hypersensitivity inhibitor. The dentifrice including the curable calcium phosphate dental cement is not particularly limited, and may contain a known additive. Examples of the known additive include foaming agents, abrasive agents (cleaning agents (excluding dibasic calcium phosphate)), moisturizers, binders (thickeners), pH adjusters, surfactants, preservatives, sweeteners (artificial sweeteners and the like), and flavors.

In the curable calcium phosphate dental cement of the present invention, the tetracalcium phosphate (A) is dissolved and reacts to be gradually converted to hydroxyapatite in the presence of water. Therefore, the first material and the second material are mixed immediately before used. The curable calcium phosphate dental cement of the present invention allows hydroxyapatite to be deposited up to a deep portion of dentinal tubule and allows the dentinal tubule to be sealed as described above. Therefore, a dentinal hypersensitivity inhibitor including the curable calcium phosphate dental cement of the present invention can be self-cured after elapse of a predetermined time and can seal the dentinal tubule. A dentinal hypersensitivity inhibitor including the curable calcium phosphate dental cement of the present invention has excellent biocompatibility, causes little skin irritation, and is excellent in safety. Furthermore, the dentinal hypersensitivity inhibitor is applied into dentinal tubule so as to rub the inhibitor, and, thereafter, excess paste can be simply removed by washing with water, and specific usage is not required. Therefore, usability is excellent. The dentinal hypersensitivity inhibitor of the present invention is not particularly limited, and may contain a known additive. Examples of the known additive include the same additives as described for the dentifrice as examples.

[Examples]

The present invention will be specifically described below by indicating examples and comparative examples. However, the present invention is not limited to these examples. The average particle diameter of particles of each of the tetracalcium phosphate (A), the alkali metal salt of phosphoric acid (B), and the acidic calcium phosphate (C) was measured on a volume basis by using a laser diffraction type particle size distribution analyzer ("Type SALD-2300" manufactured by SHIMADZU CORPORATION), and a median diameter calculated from the measurement result was set as the average particle diameter.

[Preparation of tetracalcium phosphate (A)]

Particles (average particle diameter $d_{50}$: 4.4 $\mu$m) of tetracalcium phosphate to be used as the tetracalcium phosphate (A) in Examples were obtained by pulverizing crude tetracalcium phosphate prepared as follows. Commercially available dicalcium phosphate anhydrous particles (Product No. 1430, J.T Baker Chemical Co., NJ) and calcium carbonate (Product No. 1288, J.T. Baker Chemical Co., NJ) were added to water so as to be equimolar, and the obtained product was stirred for one hour, and thereafter filtered and dried to obtain a cake-shaped equimolar mixture, and the obtained equimolar mixture was heated in an electric furnace (FUS732PB, manufactured by ADVANTEC MFS, INC) at 1500°C for 24 hours, and thereafter cooled to room temperature in a desiccator, to prepare lumps of tetracalcium phosphate. Furthermore, the lumps were roughly ground in a mortar and thereafter sieved, and, thus, fine powder and lumps of tetracalcium phosphate were removed, and the particle sizes were adjusted so as to be in a range of 0.5 to 1.0 mm, to obtain crude tetracalcium phosphate. In a 1000 ml pulverization pot ("HD-B-104 Pot Mill" manufactured by NIKKATO CORPORATION) made of alumina, 50 g of the crude tetracalcium phosphate, 200 g of zirconia balls each having a diameter of 10 mm, and 100 g of 99.5% dehydrated ethanol ("Ethanol, Dehydrated (99.5)" manufactured by FUJIFILM Wako Pure Chemical Corporation) were added, and were subjected to wet vibration pulverization at a rotation speed of 1500 rpm for 20 hours, to obtain a slurry, and ethanol was evaporated from the obtained slurry by a rotary evaporator, and the obtained product was thereafter vacuum-dried at 60°C for six hours, to obtain particles (average particle diameter $d_{50}$: 4.4 $\mu$m) of the tetracalcium phosphate (A).

[Preparation of alkali metal salt of phosphoric acid (B)]

Particles (average particle diameter $d_{50}$: 8.0 $\mu$m) of disodium hydrogen phosphate to be used as the alkali metal salt of phosphoric acid (B) in Examples were obtained as follows. In a 1000 ml pulverization pot ("HD-B-104 Pot Mill" manufactured by NIKKATO CORPORATION) made of alumina, 50 g of commercially available disodium hydrogen phosphate particles (manufactured by FUJIFILM Wako Pure Chemical Corporation), 240 g of 95% ethanol ("Ethanol (95)" manufactured by FUJIFILM Wako Pure Chemical Corporation), and 480 g of zirconia balls each having a diameter of 10 mm were added, and were subjected to wet vibration pulverization at a rotation speed of 1500 rpm for one hour, to obtain a slurry, and ethanol was evaporated from the obtained slurry by a rotary evaporator, and the obtained product was thereafter vacuum-dried at 60°C for six hours, to obtain particles (average particle diameter $d_{50}$: 8.0 $\mu$m) of disodium hydrogen phosphate.

[Preparation of acidic calcium phosphate (C)]

Dicalcium phosphate anhydrous particles (average particle diameter $d_{50}$: 1.4 $\mu$m) to be used as the acidic calcium phosphate (C) in Examples were obtained as follows. In a 1000 ml pulverization pot ("HD-B-104 Pot Mill" manufactured by NIKKATO CORPORATION) made of alumina, 50 g of commercially available dicalcium phosphate anhydrous particles (Product No. 1430, J.T Baker Chemical Co., NJ, average particle diameter of 10.2 $\mu$m), 240 g of 95% ethanol ("Ethanol (95)" manufactured FUJIFILM Wako Pure Chemical Corporation), and 480 g of zirconia balls each having a diameter of 10 mm were added, and were subjected to wet vibration pulverization at a rotation speed of 1500 rpm for 15 hours, to obtain a slurry, and ethanol was evaporated from the obtained slurry by a rotary evaporator, and the obtained product was thereafter vacuum-dried at 60°C for six hours, to obtain dicalcium phosphate anhydrous particles (average particle diameter $d_{50}$: 1.4 $\mu$m).

[Fluorine compound (E)]

The following compound was used as it was as the fluorine compound (E) to be used in Examples.
Sodium fluoride (manufactured by Sigma-Aldrich Co. LLC.)

[Organic acid (F)]

The following compounds were used as they were as the organic acid (F) to be used in Examples.

Acetic acid (manufactured by FUJIFILM Wako Pure Chemical Corporation)
Citric acid (manufactured by Tokyo Chemical Industry Co., Ltd.)
EDTA 2Na: disodium ethylenediaminetetraacetate solution (manufactured by Tokyo Chemical Industry Co., Ltd.)

[Organic acid other than (F)]

The following compounds were used as they were as an organic acid, other than (F), to be used in Examples.

85% phosphoric acid (manufactured by Tokyo Chemical Industry Co., Ltd.)
Alginic acid (molecular weight: greater than 10000, manufactured by Tokyo Chemical Industry Co., Ltd.)
The molecular weight was a weight-average molecular weight in terms of polystyrene, which was measured by gel permeation chromatography (GPC method).

[Salt of organic acid (G)]

The following compound was used as it was as the salt of organic acid (G) to be used in Examples.
Trisodium Citrate (manufactured by Tokyo Chemical Industry Co., Ltd.)

[Thickener (H)]

The following compounds were used as they were as the thickener (H) to be used in Examples.

Light anhydrous silicic acid (manufactured by NIPPON AEROSIL CO., LTD, AEROSIL (registered trademark) 130, average particle diameter: 16 $\mu$m)
Glycerin (manufactured by Tokyo Chemical Industry Co., Ltd.)
Macrogol 400 (manufactured by Yoshida Pharmaceutical Company Limited)
Macrogol 4000 (manufactured by Yoshida Pharmaceutical Company Limited)

[Preparation of curable calcium phosphate dental cement]

<Examples 1 to 8, Comparative examples 1 to 7>

(1) Preparation of first material (powder)

The composition indicated as the first material for each of examples 1 to 6 in Table 1 and comparative examples 1 to 7 in Table 2 was rotated by using a high-speed mixer (manufactured by Fukaya Kogyo K. K.) for ten minutes with an agitator at 750 rpm and a chopper at 300 rpm, to obtain powder as the first material.

(2) Preparation of first material (nonaqueous paste)

The composition indicated as the first material for each of examples 7, 8 in Table 1 was rotated by using a high-speed mixer (manufactured by Fukaya Kogyo K. K.) for 20 minutes with an agitator at 750 rpm and a chopper at 300 rpm, to obtain nonaqueous paste as the first material.

(3) Preparation of second material (liquid, aqueous paste)

The composition indicated as the second material for each of examples 1 to 8 in Table 1 and comparative examples 1 to 7 in Table 2 was stirred and dissolved by a stirrer, to obtain liquid or aqueous paste as the second material.

(4) Preparation of curable calcium phosphate dental cement paste

The first material and the second material were collected such that a mass ratio between the first material obtained in (1) or (2) and water in the second material obtained in (3) was 1.20:1.00 parts by mass, and were subjected to mixing and malaxation by a spatula for 30 seconds, to prepare the curable calcium phosphate dental cement paste. Table 1 indicates compositions of the obtained curable calcium phosphate dental cement.

[Calculation of amount in terms of fluoride ions]

The mass in terms of fluoride ions relative to the total mass in the case of the first material and the second material being mixed such that the mass ratio (the mass ratio represented as the first material/water (D) in the second material) of the first material to water in the second material was 1.20 was calculated as an amount in terms of fluoride ions (%). Table 1 and Table 2 indicate the amount in terms of fluoride ions. Fluoroapatite is considered to be formed by dentifrice containing fluoride in which the fluoride concentration is less than 0.1%, and a product is commercially available. In a case where an amount in terms of fluoride ions is greater than or equal to 0.2%, formation of fluoroapatite is expected since the concentration is higher than that of commercially available dentifrice.

(Amount in terms of fluoride ions) [%] = mass of fluoride ions contained in paste obtained by malaxation of first material and second material/(mass of first material+mass of second material) x 100

[Measurement of pH of second material]

A pH was measured by using a pH meter (LAQUA pH/IONMETER F-72; manufactured by HORIBA, Ltd.) while the second material was stirred. Table 1 and Table 2 indicate the obtained evaluation results.

[Measurement of pH of curable calcium phosphate dental cement paste]

Mixing and malaxation of the first material and the second material were performed, and the obtained product was brought into contact with pH test paper (manufactured by MACHEREY NAGEL) after elapse of 30 seconds from the start of the malaxation, to measure a pH. Table 1 and Table 2 indicate the obtained evaluation results.

[Measurement of curing time]

The first material and the second material were collected such that a mass ratio (mass ratio represented as the first material/water (D) in the second material) of the first material to water in the second material was 1.20, and were thereafter subjected to mixing and malaxation by a spatula for 30 seconds. A $\varphi$10 mm mold having a thickness of 1 mm was filled with paste obtained after the malaxation so as to slightly spill the paste, a glass cover was placed, and a flat plate was brought into pressure contact therewith. Thereafter, the glass cover was removed, and the obtained product was put in a condition of 37°C and 95%RH within four minutes after the start of the malaxation. Every ten minutes from the start of the malaxation, an indenter (mass: 100g, needle: $\varphi$1 mm) was slowly moved downward to and pushed into the surface of the product obtained by the malaxation. At this time, the shortest time during which an indentation was not confirmed on the surface of the product obtained by the malaxation and which was measured from the start of the mixing and malaxation of the first material and the second material was set as a curing time (n=1). Table 1 and Table 2 indicate the obtained evaluation results. The curing time is required to be longer than or equal to five minutes, considering that the product obtained by the malaxation is applied into dentinal tubule so as to rub the product when used as a hypersensitivity inhibitor. The calcium phosphate cement is required to be held until the cured product is settled at a desired position, and the curing time needs to be shorter than or equal to 45 minutes, and preferably shorter than or equal to 40 minutes form the practical viewpoint.

[Evaluation of sealability for dentinal tubule]

(1) Preparation of bovine tooth for evaluating sealability for dentinal tubule

A cheek-side center of a healthy bovine incisor tooth was ground and trimmed with #80, #1000 sand papers by using a rotary grinder, to produce a 2 mm thick dentin plate at which the cheek-side dentin was exposed. The cheek-side dentin surface was further ground with lapping films (#1200, #3000, #8000, manufactured by Sumitomo 3M Ltd.) so as to be smooth. In this cheek-side dentin portion, a window of a test portion over 2 mm in each of the ordinate direction and the abscissa direction with respect to the tooth was left. The bovine tooth was subjected to ultrasonic wave for ten minutes and the dentin window was demineralized while the bovine tooth was immersed in 3% EDTA solution, and the bovine tooth was thereafter washed with water to prepare a bovine tooth used for evaluating sealability for dentinal tubule. A sufficient amount of the curable calcium phosphate dental cement paste of the present invention which was prepared as described above was adhered to the cheek-side dentin surface of the bovine tooth, and was applied onto the entire surface of the dentin window so as to rub the paste by using a micro brush ("REGULAR SIZE (2.0 mm), MRB400" manufactured by MICROBRUSH INTERNATIONAL) for 30 seconds. Thereafter, the paste on the dentin surface was removed with distilled water (n=3).

(2) Preparation of sample for SEM observation

After the above-described treatment, the bovine tooth was dried by air-blowing, to obtain a sample for observing dentinal tubule.

(3) SEM observation

For SEM observation, an SU-3500 (manufactured by Hitachi High-Technologies Corporation) was used. Morphology was observed on the dentin surface to which the curable calcium phosphate dental cement paste had not been applied, and on the bovine tooth surface at the time when the paste was applied at an accelerating voltage of 5 kV (n=3). One sample for SEM observation was observed at any three points, and sealability for dentinal tubule was represented by the following equation.

$$\text{Dentinal tubule sealing rate (\%)} = \text{sealed dentinal tubule (the number thereof)/observed dentinal tubule (the number thereof)} \times 100$$

The average of the dentinal tubule sealing rates of three samples was evaluated based on the following criteria. Table 1 and Table 2 indicate the obtained evaluation results.

Excellent: The dentinal tubule sealing rate was greater than or equal to 80%.
Good: The dentinal tubule sealing rate was greater than or equal to 40% and less than 80%.
Poor: The dentinal tubule sealing rate was less than 40%.

[Storage stability]

In example 5, after the second material was prepared, the second material was put in a bottle made of polyethylene, and immediately stored in a condition of 25°C, and presence or absence of precipitation was visually confirmed after 24 hours (n=1). No precipitation was confirmed, and it was confirmed that the storage stability was excellent.

[Table 1]

| Component (parts by mass) | | | Ex. 1 | Ex. 2 | Ex. 3 | Ex. 4 | Ex. 5 | Ex. 6 | Ex. 7 | Ex. 8 |
|---|---|---|---|---|---|---|---|---|---|---|
| First material | Tetracalcium phosphate (A) | Tetracalcium phosphate | 69.8 | 67.0 | 63.4 | 67.0 | 69.6 | 35.1 | 5.7 | 10.2 |
| | Alkali metal salt of phosphoric acid (B) | Disodium hydrogen phosphate | 11.6 | 11.2 | 10.6 | 11.2 | 11.6 | 5.9 | 0.9 | 1.7 |
| | Acidic calcium phosphate (C) | Dicalcium phosphate anhydrous | 23.3 | 22.3 | 21.1 | 22.3 | 23.2 | 11.7 | 1.9 | 3.4 |
| | Thickener (H) | Light anhydrous silicic acid | 11.6 | 11.2 | 10.6 | 11.2 | 11.6 | 5.9 | 0.9 | 1.7 |
| | | Glycerin | | | | | | | 25.1 | 45.3 |
| | | Macrogol 400 | | | | | | | 16.4 | 29.6 |
| | | Macrogol 4000 | | | | | | | 7.6 | 13.7 |
| Second material | Water (D) | Purified water | 97.0 | 93.0 | 88.0 | 93.0 | 96.7 | 48.8 | 48.8 | 88.0 |
| | Fluorine compound (E) | Sodium fluoride | 2.0 | 2.0 | 2.0 | 2.0 | 1.0 | 2.0 | 2.0 | 2.0 |
| | Organic acid (F) | EDTA-2Na | | | | 5.0 | | | | |
| | | Citric acid | 1.0 | 5.0 | 10 | | 0.02 | 10.0 | 10.0 | 10.0 |
| | Salt of organic acid (G) | Trisodium Citrate | | | | | 2.24 | | | |
| | Organic acid other than (F) | 85% phosphoric acid | | | | | | | | |
| | | Alginic acid | | | | | | | | |
| | Thickener (H) | Glycerin | | | | | | 28.0 | 28.0 | |
| | | Macrogol 400 | | | | | | 13.0 | 13.0 | |
| | | Macrogol 4000 | | | | | | 0.2 | 0.2 | |
| Amount in terms of fluoride ions (%) | | | 0.44 | 0.45 | 0.46 | 0.45 | 0.22 | 0.59 | 0.59 | 0.46 |
| Organic acid (F)/entirety of cement (%) | | | 0.46 | 2.4 | 4.9 | 2.4 | 0.01 | 6.2 | 6.2 | 4.9 |
| Organic acid (F)+salt of organic acid (G)/entirety of cement (%) | | | 0.46 | 2.4 | 4.9 | 2.4 | 1.0 | 6.2 | 6.2 | 4.9 |
| pH of second material | | | 5.37 | 3.88 | 3.03 | - | 7.00 | - | - | - |
| pH of paste immediately after malaxation of first material and second material | | | 8.8 | 7.0 | 5.5 | 8.5 | 8.8 | 8.2 | 7.3 | 7.6 |

EP 4 268 794 A1

(continued)

| Component (parts by mass) | Ex. 1 | Ex. 2 | Ex. 3 | Ex. 4 | Ex. 5 | Ex. 6 | Ex. 7 | Ex. 8 |
|---|---|---|---|---|---|---|---|---|
| Curing time (minute) | 40 | 30 | 10 | 40 | 30 | 30 | 30 | 30 |
| Dentinal tubule sealing rate (%) | Excellent | Excellent | Excellent | Excellent | Excellent | Good | Good | Good |
| Mass ratio represented as first material/water (D) in second material during malaxation of first material and second material | 1.20 | 1.20 | 1.20 | 1.20 | 1.20 | 1.20 | 1.20 | 1.20 |

[Table 2]

| Component (parts by mass) | | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| First material | Tetracalcium phosphate (A) | Tetracalcium phosphate | 70.6 | 71.3 | 71.8 | 70.6 | 70.4 | 63.4 | 69.8 |
| | Alkali metal salt of phosphoric acid (B) | Disodium hydrogen phosphate | 11.8 | 11.9 | 12.0 | 11.8 | | 10.6 | 11.6 |
| | Acidic calcium phosphate (C) | Dicalcium phosphate anhydrous | 23.5 | 23.8 | 23.9 | 23.5 | 23.5 | 21.1 | 23.3 |
| | Thickener (H) | Light anhydrous silicic acid | 11.8 | 11.9 | 12.0 | 11.8 | 11.7 | 10.6 | 11.6 |
| | | Glycerin | | | | | | | |
| | | Macrogol 400 | | | | | | | |
| | | Macrogol 4000 | | | | | | | |
| Second material | Water (D) | Purified water | 98.0 | 99.0 | 99.8 | 98.0 | 88.0 | 88.0 | 97.0 |
| | Fluorine compound (E) | Sodium fluoride | 2.0 | 1.0 | 0.25 | 2.0 | 2.0 | 2.0 | 2.0 |
| | Organic acid (F) | EDTA-2Na | | | | | | | |
| | | Citric acid | | | | | 10.0 | | |
| | Salt of organic acid (G) | Trisodium Citrate | | | | | | | |
| | Organic acid other than (F) | 85% phosphoric acid | 1.1 | | | | | | 1.0 |
| | | Alginic acid | | | | | | 10.0 | |
| | Thickener (H) | Glycerin | | | | | | | |
| | | Macrogol 400 | | | | | | | |
| | | Macrogol 4000 | | | | | | | |
| Amount in terms of fluoride ions (%) | | | 0.43 | 0.22 | 0.05 | 0.44 | 0.46 | 0.46 | 0.43 |
| Organic acid (F)/entirety of cement (%) | | | - | - | - | - | 4.9 | - | - |
| Organic acid (F)+salt of organic acid (G)/entirety of cement (%) | | | - | - | - | - | 4.9 | - | - |
| pH of second material | | | 4.03 | 9.05 | 8.19 | 8.69 | - | - | 4.17 |
| pH of paste immediately after malaxation of first material and second material | | | 8.5 | 8.8 | 8.8 | 11 | - | - | 8.5 |

(continued)

| Component (parts by mass) | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|
| Curing time (minute) | 50 or longer | 50 or longer | 50 or longer | 50 or longer | 40 | 50 or longer | 50 or longer |
| Dentinal tubule sealing rate (%) | Excellent | Excellent | Excellent | Excellent | Poor | - | Excellent |
| Mass ratio represented as first material/water (D) in second material during malaxation of first material and second material | 1.20 | 1.20 | 1.20 | 1.20 | 1.20 | 1.20 | 1.20 |

As is apparent from the above-described results, the curable calcium phosphate dental cement according to the present invention (examples 1 to 8) included the fluorine compound (E) that was expected to enhance acid resistance, had sealability for dentinal tubule, and allowed a curing time to be adjusted such that the curing time was shorter than or equal to 40 minutes. Furthermore, it was indicated that, in the curable calcium phosphate dental cement according to the present invention (examples 1 to 8), the sealability for dentinal tubule was greater than or equal to 40%, and the sealability for dentinal tubule was higher than the dentinal tubule sealing rate of less than 40% in the curable calcium phosphate dental cement (comparative example 5) which did not include particles of alkali metal salt of phosphoric acid. Furthermore, in the second material according to example 5 of the present invention in which the salt of organic acid (G) was added to the second material and the pH of the second material was greater than or equal to 6.0, it was indicated that precipitation was not generated at 25°C after 24 hours, and storage stability was excellent. It was indicated that the curable calcium phosphate dental cement (example 3) that included the organic acid (F) having a molecular weight of 10000 or less had a curing time shorter than that of the curable calcium phosphate dental cement (comparative example 6) that included an organic acid having a molecular weight of greater than 10000.

As described above, comparison between comparative example 6 corresponding to Patent Literature 1 and example 3 indicates that the curable calcium phosphate dental cement according to the present invention exhibited advantageous effects that a curing time was significantly shortened and sealability for dentinal tubule was excellent. Moreover, the curable calcium phosphate dental cement according to the present invention was expected to form fluoroapatite, and enhancement of acid resistance was thus expected.

According to Patent Literature 3, by mixing sodium fluoride with calcium phosphate cement powder, the concentration of calcium released in a solution is reduced, and the concentration of phosphate ions is relatively increased, and the increase of the concentration of phosphate ions promotes cement setting reaction. That is, it is suggested that the curing time is shortened by containing sodium fluoride. However, unlike suggestion of Patent Literature 3, as indicated in comparative examples 1 to 4 and 6 to 7, it was confirmed that, in a case where the first material that was calcium phosphate cement powder and the second material including sodium fluoride were mixed, calcium fluoride was generated, and the concentration of calcium was reduced in the entire system, and, thus, the curing reaction was delayed, and the curing time was increased.

INDUSTRIAL APPLICABILITY

The curable calcium phosphate dental cement according to the present invention is advantageously used as a dentinal hypersensitivity inhibitor and dentifrice in the field of dentistry.

**Claims**

1.    Curable calcium phosphate dental cement comprising:

     a first material as powder or nonaqueous paste; and
     a second material as liquid or aqueous paste, wherein
     the first material comprises tetracalcium phosphate (A), alkali metal salt of phosphoric acid (B), and acidic calcium phosphate (C),
     the second material comprises water (D), and
     at least one of the first material and the second material comprises a fluorine compound (E) and an organic acid (F) having a molecular weight of 10000 or less.

2.    The curable calcium phosphate dental cement according to claim 1, wherein the fluorine compound (E) is at least one selected from the group consisting of sodium fluoride, potassium fluoride, sodium monofluorophosphate, and stannous fluoride.

3.    The curable calcium phosphate dental cement according to claim 1 or 2, wherein the fluorine compound (E) is sodium fluoride or potassium fluoride.

4.    The curable calcium phosphate dental cement according to any one of claims 1 to 3, wherein an amount of the fluorine compound (E) in terms of fluoride ions is 0.01 to 3% of an entire amount of the curable calcium phosphate dental cement.

5.    The curable calcium phosphate dental cement according to any one of claims 1 to 4, wherein the organic acid (F) having a molecular weight of 10000 or less is a polyvalent organic acid having two or more acidic groups.

6. The curable calcium phosphate dental cement according to any one of claims 1 to 5, wherein the organic acid (F) having a molecular weight of 10000 or less is at least one selected from the group consisting of citric acid, malonic acid, succinic acid, oxalic acid, malic acid, and tartaric acid.

7. The curable calcium phosphate dental cement according to any one of claims 1 to 6, wherein the organic acid (F) having a molecular weight of 10000 or less is citric acid.

8. The curable calcium phosphate dental cement according to any one of claims 1 to 7, wherein a content of the organic acid (F) having a molecular weight of 10000 or less is greater than or equal to 0.005 parts by mass with respect to 100 parts by mass of the entire amount of the curable calcium phosphate dental cement.

9. The curable calcium phosphate dental cement according to any one of claims 1 to 8, wherein at least one of the first material and the second material further comprises salt of organic acid (G) having a molecular weight of 10000 or less.

10. The curable calcium phosphate dental cement according to any one of claims 1 to 9, wherein at least one of the first material and the second material further comprises at least one selected from the group consisting of metal oxide particles and light anhydrous silicic acid particles which have an average particle diameter of 0.002 to 20 $\mu$m.

11. The curable calcium phosphate dental cement according to any one of claims 1 to 10, wherein the second material has a pH of greater than or equal to 6.0.

12. The curable calcium phosphate dental cement according to any one of claims 1 to 11, wherein paste immediately after malaxation of the first material and the second material has a pH of greater than or equal to 5.5.

13. The curable calcium phosphate dental cement according to any one of claims 1 to 12, wherein the second material comprises the fluorine compound (E), and the organic acid (F) having a molecular weight of 10000 or less.

14. The curable calcium phosphate dental cement according to any one of claims 1 to 13, wherein the first material is powder, and the second material is liquid.

15. The curable calcium phosphate dental cement according to any one of claims 1 to 14, wherein the tetracalcium phosphate (A) is in a form of particles, and has an average particle diameter of 0.5 to 40 $\mu$m.

16. The curable calcium phosphate dental cement according to any one of claims 1 to 15, wherein the alkali metal salt of phosphoric acid (B) is in a form of particles, and has an average particle diameter of 0.5 to 20 $\mu$m.

17. The curable calcium phosphate dental cement according to any one of claims 1 to 16, wherein the acidic calcium phosphate (C) is in a form of particles, and has an average particle diameter of 0.1 to 7 $\mu$m.

18. Dentifrice comprising the curable calcium phosphate dental cement according to any one of claims 1 to 17.

19. A dentinal hypersensitivity inhibitor comprising the curable calcium phosphate dental cement according to any one of claims 1 to 17.

## INTERNATIONAL SEARCH REPORT

| International application No. |
| --- |
| **PCT/JP2021/048984** |

**A. CLASSIFICATION OF SUBJECT MATTER**

*A61K 6/853*(2020.01)i; *A61K 6/864*(2020.01)i
FI:   A61K6/864; A61K6/853

According to International Patent Classification (IPC) or to both national classification and IPC

**B. FIELDS SEARCHED**

Minimum documentation searched (classification system followed by classification symbols)

A61K6/853; A61K6/864

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2010/113800 A1 (KURARAY MEDICAL INC.) 07 October 2010 (2010-10-07)<br>    claims 1-12, examples 1-24 | 1-19 |
| Y | JP 62-83348 A (KABUSHIKI KAISHA ADVANCED RESEARCH INSTITUTE) 16 April 1987 (1987-04-16)<br>    tables 3, 4 | 1-19 |
| Y | 澤村武憲. リン酸カルシウム骨ペーストの硬化特性に対する影響因子に関する研究. 名古屋工業大学学術機関リポジトリ. 2016, Internet: <URL: http://doi.org/10.20602/00003239>, (SAWAMURA, Takenori. Improving the setting properties of calcium phosphate cements. Nagoya Institute of Technology Repository System.)<br>    p. 3, section 1.2, p. 23, section 2.5, p. 38, section 4.1, p. 50, section 4.3.2 | 1-19 |
| Y | WANG, X. et al. Effects of additives on the rheological properties and injectability of a calcium phosphate bone substitute material. J Biomed Mater Res B Appl Biomater. 2005, 78B(2), 259-264<br>    tab. I, fig. 8, 11, 12 | 1-19 |

☐ Further documents are listed in the continuation of Box C. ☑ See patent family annex.

| * | Special categories of cited documents: |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **24 February 2022** | **29 March 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2021/048984**

| Patent document cited in search report | Publication date (day/month/year) | Patent family member(s) | Publication date (day/month/year) |
|---|---|---|---|
| WO 2010/113800 A1 | 07 October 2010 | US 2012/0027829 A1 claims, examples <br> EP 2425843 A1 | |
| JP 62-83348 A | 16 April 1987 | (Family: none) | |

Form PCT/ISA/210 (patent family annex) (January 2015)

**EP 4 268 794 A1**

REFERENCES CITED IN THE DESCRIPTION

**Patent documents cited in the description**

- WO 2010113800 A
- US 8557038 B
- JP 2007522113 A

23